Europäisches Patentamt

European Patent Office

Office européen des brevets

(11) Publication number: **0 198 327 B1**

## EUROPEAN PATENT SPECIFICATION

(45) Date of publication of patent specification: **03.03.93** (51) Int. Cl.⁵: **G01N 21/82**, G01N 33/53

(21) Application number: **86104495.6**

(22) Date of filing: **02.04.86**

The file contains technical information submitted after the application was filed and not included in this specification

(54) **Method of automatically detecting agglutination reaction and apparatus therefor.**

(30) Priority: **03.04.85 JP 70403/85**

(43) Date of publication of application:
**22.10.86 Bulletin  86/43**

(45) Publication of the grant of the patent:
**03.03.93 Bulletin  93/09**

(84) Designated Contracting States:
**BE CH DE FR GB LI NL SE**

(56) References cited:
**EP-A- 0 129 450**
**US-A- 4 197 088**

**PATENT ABSTRACTS OF JAPAN, vol. 8, no. 223 (P-307)[1660], 12th October 1984; & JP-A-59 105 543**

(73) Proprietor: **GREEN CROSS CORPORATION 15-1, Imabashi-1-chome Higashi-ku Osaka-shi**
**Osaka 541(JP)**

(72) Inventor: **Inoue, Yoshiyuki**
**A37-301, Otokoyama Koro 6-banchi**
**Yawata-shi Kyoto(JP)**
Inventor: **Yoshimura, Kazuto**
**582, Soga-cho**
**Kashihara-shi Nara(JP)**
Inventor: **Mimura, Masanobu**
**6-3-9, Uozakikitamachi Higashinada-ku**
**Kobe-shi Hyogo(JP)**
Inventor: **Shiino, Satoru**
**39-333, Kohata Okurayama**
**Uji-shi Kyoto(JP)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40 (DE)**

EP 0 198 327 B1

Rank Xerox (UK) Business Services
(3.10/3.5x/3.0.1)

**Description**

This invention relates to a method of detecting a specific immune substance occurring in a sample and an apparatus for carrying out said method.

The technique of detecting an antigen or antibody by utilizing the agglutination reaction, in particular the one using tannic acid-treated erythrocytes, is in wide use in the field of clinical examination since the detection sensitivity is 100-1,000 times higher as compared with the one-dimensional immunodiffusion, two-dimensional immunodiffusion or crossed immunoelectrophoretic technique.

Thus, said technique is widely used, for instance, in assaying anti-HBs antibody using HBs antigen-sensitized erythrocytes [Nagase et al.: Medical Postgraduates, 16 (4), 13 (1974); Tateda et al.: Kiso to Rinsho (The Clinical Report), 11 (7), 2091 (1977) ], in detecting HBs antigen using anti-HBs antibody-sensitized erythrocytes [Taguchi et al.: Rinsho Kensa (Journal of Medical Technology), 22 (4), 437 (1978)] , and in sensitizing tetanus antitoxin with tetanus toxin-sensitized erythrocytes followed by screening antitetanus antitoxin antibody using the thus-sensitized erythrocytes [A. Barr et al.: Journal of Clinical Pathology, 28, 969 (1975) ].

Whereas such antigen or antibody detection methods based on the agglutination reaction are in wide application owing to their high detection sensitivity, said methods also have a serious drawback that the error in judgement is not slight because whether there is a difference or not between the agglutination picture of a negative control and that of a sample on a microplate is judged visually.

JP-A-59-105543 discloses a method and device for the detection of the degree of agglutination, generated by an antigen-antibody reaction, wherein the agglutinate lumps formed on a plate are irradiated with infrared light from below, and the reaction surface is scanned by a line sensor arranged above the plate to detect the particles.

US-A-4 197 088 discloses a method and apparatus for the qualitative and quantitative determination of immunological reactions, wherein the reaction zone containing agglutinate particles is transilluminated and the transmitted light is imaged on an image sensor. The total dark area formed on the surface of the image sensor is measured electronically and is compared with the total dark area obtained by using a reference specimen.

An object of the invention is to provide a method of detecting an agglutination reaction with only minor errors of judgment.

Another object of the invention is to provide a testing apparatus capable of automatically and quantitatively determining a specific immune substance occuring in a sample.

The invention thus provides a method of quantitatively assaying an antigen or antibody in a sample, which comprises

(1) mixing the antigen- or antibody-containing sample and a support for agglutination reaction as sensitized with an antibody or antigen, respectively, corresponding to the antigen or antibody in said sample, in a reaction vessel to carry out an agglutination reaction, wherein the shape of the reaction vessel bottom is a spherical or conical one,

(2) allowing the agglutinate formed in step (1) to precipitate on the bottom of the reaction vessel to form an agglutinate precipitate,

(3) passing light from a light source through the reaction vessel from the bottom or the top thereof, while generating an intensity of the light after its passage through the agglutinate precipitate,

(4) continuously sensing the intensity of the transmitted light on a line passing the center of the agglutinate precipitate with a one-dimensional image sensor, and

(5) outputting the sensed result as a sensor output wave form (shown by voltage) and then measuring the diameter of the agglutinate precipitate in terms of the sensor output wave form width at a voltage intermediate between the standard voltage found with a negative control and the voltage found with the sample.

Furthermore, the invention provides an apparatus suitable for quantitatively assaying an antigen or antibody in a sample, which comprises

(a) a reaction vessel having a spherical or conical bottom for mixing the antigen- or antibody-containing sample and a support for agglutination reaction as sensitized with an antibody or antigen, respectively, corresponding to the antigen or antibody in said sample to carry out an agglutination reaction, and then allowing the agglutinate formed to precipitate on the bottom of said reaction vessel to form an agglutinate precipitate,

(b) a light source comprising a reflector and a halogen lamp,

(c) a means for passing a light from said light source through the reaction vessel from the bottom or the top thereof, while generating an intensity of the light after its passage through the agglutinate precipitate, comprising a mirror, diffusion plate for uniform light, vessel-plate-supporting and -transferring frame and a sample-carrying plate,

(d) a means for continuously sensing the intensity of the transmitted light on a line passing the center of the agglutinate precipitate with a one-dimensional image sensor, comprising a mirror, an optical lens and a CCD linear image sensor,

(e) a means for measuring the diameter of the agglutinate precipitate by outputting the result sensed by said means (d) as a sensor wave form (shown by voltage) and in terms of the sensor output wave form width at a voltage intermediate between the standard voltage found with a negative control and the voltage found with the sample, comprising a A/D converter unit and a sensor wave form signal memory buffer, and

(f) a means for calculating the size of the agglutinate precipitate by operation treatment of the value as measured by said means (e) for evaluation and judgment, comprising an operation panel unit, a wave form-processing computer unit and a printer.

In the accompanying drawings,

Fig. 1 shows typical examples of the output wave form as obtained by a one-dimensional image sensor;

Fig. 2 is an output wave form as obtained with a positive control;

Fig. 3 are enlarged views each of a part of an output wave form, the wave form (a) being a sensor output wave form for a negative control and the wave form (b) being a sensor output wave form for a positive sample;

Fig. 4 shows graphically and schematically the correlation between the precipitate diameter and the five-step scoring employed; and

Fig. 5 is a schematic representation of an apparatus in accordance with an embodiment of the invention.

In the figures, the numerical indices respectively indicate the following:

(1)     Output wave form for a buffer alone
(2)     Output wave form for a negative control
(3)     Output wave form for a positive sample
F       Picture (agglutinate precipitate)
1       Reflector
2       Halogen lamp
3       Mirror
4       Diffusion plate for uniform light
5       Vessel-plate-supporting and -transferring frame
6       Sample-carrying plate
7       Mirror
8       Optical lens
9       CCD linear image sensor
10      A/D converter unit
11      Sensor wave form signal memory buffer
12      Operation panel unit
13      Wave form-processing computer unit
14      Printer

(a) Light source and sample vessel

Natural or monochromatic light is most suited as the light source to be used in the practice of the invention. The wavelength to be used is preferably 300-800 nm. The light from the light source enters the sample vessel either from the bottom or from the top thereof. When a halogen lamp is used, the quantity of light which gives good results is such that the electric power consumption amounts to 1 watt to 1 kilowatt. The shape of the sample vessel bottom is preferably, but not particularly limited to, a spherical or conical one. The transverse cross-sectional side view of the sample vessel is generally circular, or triangular, square or polygonal. It is convenient for the procedure and measurement that the inside diameter is 3-20 mm.

Any material which is light-transmitting and inert may be used for constructing reaction vessels, but glass acrylic resins, vinyl choride resins and styrene resins are preferred. In particular, styrene resins are most preferred in view of the stability of the agglutination picture. However, since such stability can be increased by employing an appropriate shape of the vessel bottom, for example the spherical or inclined type or some other suited for the material used, the material is not necessarily limited to a styrene resin.

3

EP 0 198 327 B1

(b) Agglutination reaction

The support for agglutination reaction to be used in the practice of the invention includes the so-far known latex resins, animal erythrocytes (e.g. of sheep, guinea pig, human of the blood group O, or chicken) denatured by treatment with formalin, glutaraldehyde, tannic acid, etc., and so forth. Preferred are denatured animal erythrocytes.

This support for agglutination reaction is used for sensitization with an antibody or antigen generally and preferably in the form of a suspension in an aqueous medium. The aqueous medium is, for example water, physiological saline, a buffer (phosphate buffer, glycine buffer, borate buffer, etc.) or the like.

Generally, the support for agglutination reaction is dispersed in an aqueous medium in an concentration of about 0.1-10% (by volume) and the suspension is preferably adjusted to pH about 7.0-8.9, in particular about 7.2-8.0. The sensitization of this support for agglutination reaction can be performed in accordance with a per se known method [Igaku no ayumi, 78, 759-760 (1970) ]. The sensitization is preferably carried out in an aqueous medium such as mentioned above and can be effected by mixing an antigen or antibody suspension with the support for agglutination reaction.

The sensitization reaction is carried out generally at a pH of 4.5-8.6 and a temperature of about 15-37 °C for about 15-60 minutes.

The concentration of and proportion between the antigen or antibody suspension and the support for agglutination reaction both to be subjected to reaction should be selected so as to be optimal depending on the kind of antigen or antibody suspension and the kind of support for agglutination reaction. For instance, when a 5% suspension of glutaraldehyde-treated sheep erythrocytes and an HBs antigen suspension are used, it is preferable to adjust the HBs antigen concentration to 0.015-0.15 in terms of the reading of absorbance ( $E_{280}$ value) and add thereto glutaraldehyde-treated sheep erythrocytes (5% suspension) in an amount of 1-11 times as large.

After attaining sensitization in this manner, the sensitized support for agglutination reaction is washed and then suspended in an appropriate concentration, and the suspension is distributed in appropriate portions and lyophilized. For use as a reagent for agglutination reaction assaying, the lyophilizate is diluted with an appropriate aqueous medium in the above-mentioned reaction vessel.

The proportion between the sensitized support for agglutination reaction and the sample in carrying out an agglutination reaction in the reaction vessel specifically described herein may be selected according to the conventional practice with agglutination reaction reagents for qualitative determination. In the case of sensitized erythrocytes, for instance, a suspension of the sensitized erythrocytes in a concentration of 0.5 ± 0.1% is mixed by shaking with an equal volume of a sample. Although the sample and the sensitized erythrocytes suspension volume are not particularly limited or critical, it is economical to mix them in equal volumes of 0.1-0.5 ml.

The time from mixing of sensitized erythrocytes with a sample in the above-mentioned reaction vessel to formation of an agglutination picture may vary to some extent depending on the kind of the erythrocytes used. Generally, however, a distinct agglutination image appears in 1-2 hours of allowing the reaction vessel to stand vertically at room temperature and, therefore, judgement may be made within the subsequent 24 hours. The same applied also to the case in which a conventional support in general use for agglutination reactions, such as a latex, is used in lieu of the erythrocytes to serve as a support in sensitization.

(c) Measurement of agglutinate precipitate

The antigen or antibody in sample is quantitatively determined by allowing the agglutinate formed in the reaction vessel to precipitate on the bottom of the reaction vessel and measuring the diameter of the resulting agglutinate precipitate by means of one-dimensional image sensor capable of sensing, on a line passing the center of said agglutinate precipitate, the intensity of the light emitted from a light source after its passage through the agglutinate precipitate.

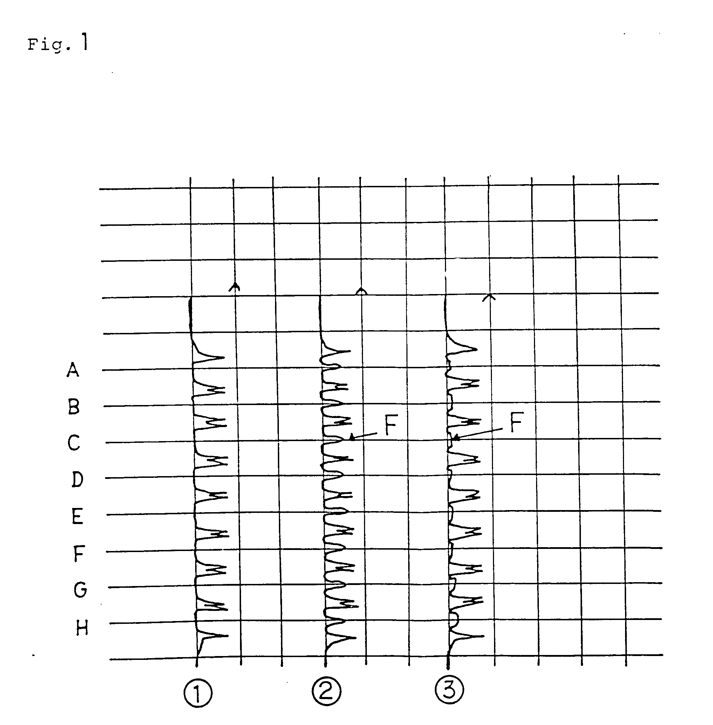

Fig. 1 shows a typical output wave form for an agglutinate precipitate as obtainable with a one-dimensional image sensor. In the figure, wave form (1) is the output wave form for a buffer alone, wave form (2) is the one for a negative control and wave form (3) is the one for a positive sample.

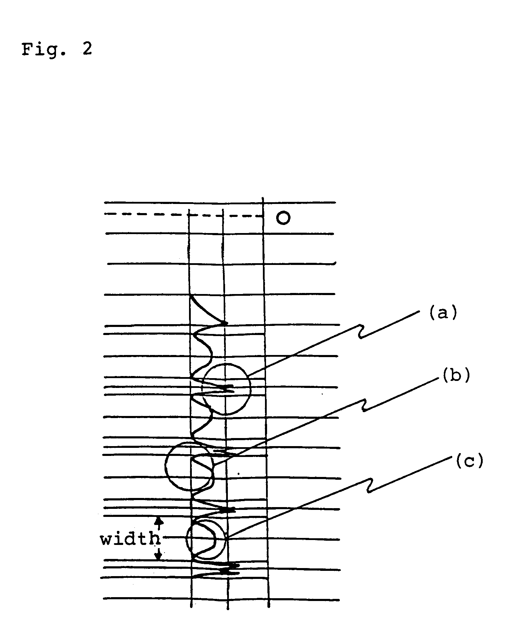

Fig. 2 shows a wave form for a positive sample, part (a) of the wave form being characteristic of the space between neighboring wells on the vessel. Part (b) indicates the well (sample vessel) edge. The depression in part (c) is due to an agglutinate precipitate, the wave form width and potential difference varying depending on the positivity, negativity and so on.

The more positive the sample is, the broader the wave form width is and the larger the potential difference is. The converse applies in negative cases.

4

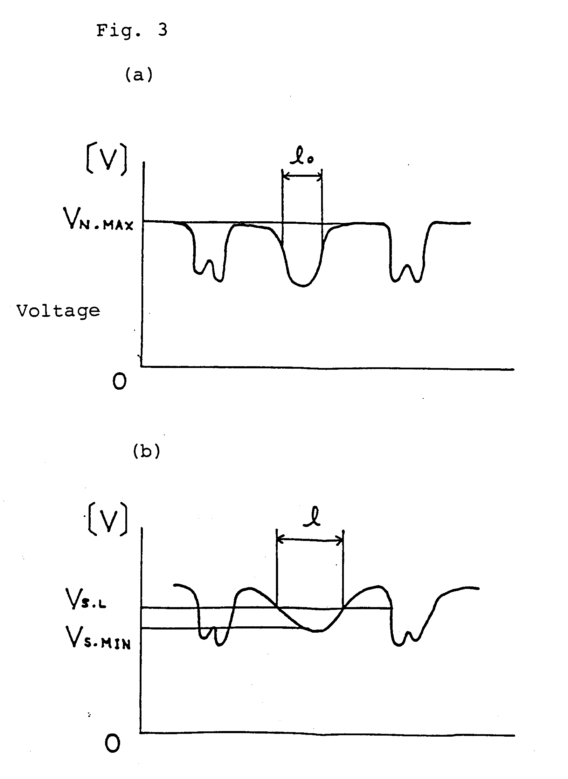

Fig. 3 shows enlarged views each of a part of an output wave forms. In Fig. 3, wave form (a) is the sensor output wave form for a negative control, $V_{N.\ MAX}$ being the voltage on both well edges whereas wave form (b) is the line sensor output wave form for a positive sample, $V_{S.L.}$ being the reference voltage for diameter measurement and $V_{S.\ MIN}$ being the minimum wave form voltage. The output wave form varies in the gradient of voltage change among the sample, positive and negative controls. Therefore, for uniformization of measurement conditions, a voltage $V_{S.L.}$ is determined which is intermediate between the reference voltage for a buffer in a well (voltage on both well edges, $V_{N.\ MAX}$) as found in the sensor output wave form for a negative control and the minimum wave form voltage $V_{S.\ MIN}$ for each sample. For instance, when the intermediate value is taken at a level corresponding to 1/2 of the difference between both voltages, the voltage value in question is calculated as follows;

$$V_{S.L.} = (V_{N.\ MAX} - V_{S.\ MIN}) \times 1/2 + V_{S.\ MIN}$$

The wave form width $\ell$ which corresponds to this standard voltage value for diameter measurement is regarded as the diameter for the sample in question. This method makes it possible to absorb the dispersion resulting from different lots of agglutinate precipitate and achieve uniformization of measurement conditions.

(d) Rating based on the agglutinate precipitate diameter

The diameter ($\ell$) obtained above in (c) is evaluated quantitatively and rated by constructing a calibration curve or working curve by increasing the antigen quantity in positive sample against a negative control. Generally employed is the method based on the rate of increase in $\ell$ or the one based on comparison with empirical data.

(i) Method based on the rate of increase in $\ell$

The diameter for a positive control is rated as "4" and that for a negative control as "0". The diameter for a sample, which is within this range, is rated based on the ratio of the diameter difference between the sample and the negative control to the diameter difference between the positive and the negative control, the latter difference being regarded as 1.

$$d\ell = \ell - \ell_0 / \ell_4 - \ell_0$$

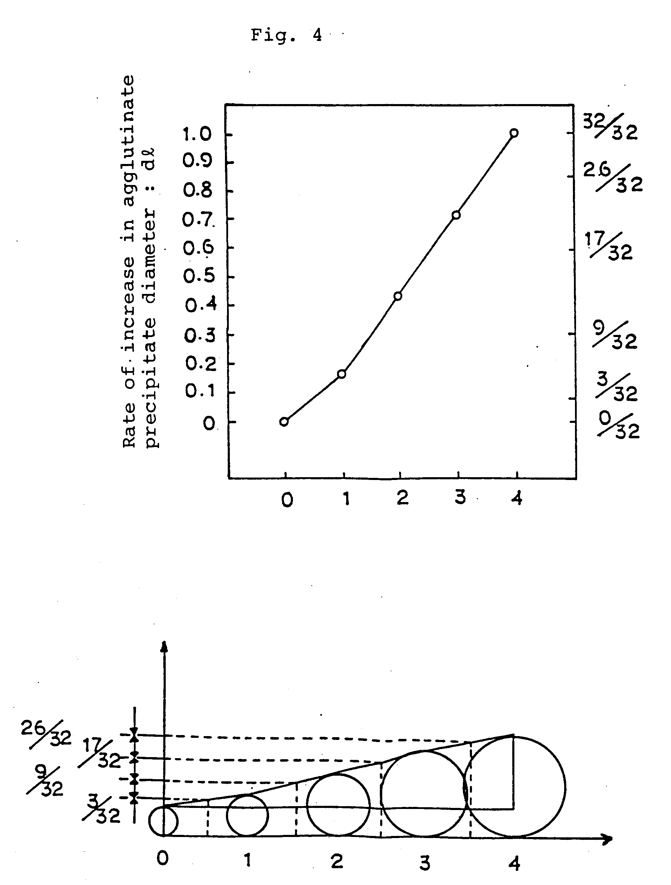

where
$\ell$ is the wave form width for the sample,
$\ell_0$ is the width for the negative control, and
$\ell_4$ is the width for the positive control.
This relation is as shown in fig. 4, or in Table 1.

## Table 1

### Criteria for judging agglutinate image diameter increases

| image diameter increases | Rating |
|---|---|
| $0 \leqq d\ell \leqq 3/32$ | 0 |
| $3/32 < d\ell \leqq 9/32$ | 1 |
| $9/32 < d\ell \leqq 17/32$ | 2 |
| $17/32 < d\ell \leqq 26/32$ | 3 |
| $26/32 < d\ell \leqq 1$ | 4 |

(ii) Method based on comparison with known data

According to the empirical values reported by persons engaged in judging the results of agglutination reaction tests, the evaluation result scores correspond to the respective agglutinate diameter value ranges given below in Table 2.

## Table 2

### Criteria for judging agglutinate

| image diameters | Rating |
|---|---|
| 1.4 mm to 1.9 mm | 0 |
| 2.0 mm to 2.4 mm | 1 |
| 2.5 mm to 3.0 mm | 2 |
| 3.1 mm to 3.7 mm | 3 |
| 3.8 mm or more | 4 |

The values for rating as given above in (a) and (b) are experience-based values. They may be modified as necessary, however.

The contents of the above calibration curve or rating are programmed in a computer to thereby operate a judging device. The basic algorithm for judgement which constitutes the skeleton of the program is given below.

Start

○ ←

↓

〔Sampling of a negative control〕

↓

〔Sampling of a positive control〕

↓

〔Sampling of a material to be assayed〕

↓

〔Calculation of the diameter of each blood

cell image and 5-score rating〕

↓

〔Calibration according to the principle

of dilution in the 5-score rating〕

↓

〔Printing out of the results 〕

Example 1

A 0.5% suspension of anti-HBs antibody-sensitized sheep erythrocytes [ANTIHEBSCELL® (available from The Green Cross Corp.)] was distributed in 0.25 ml portions into polystyrene test tubes (capacity 14 ml; the bottom inside surface having a radium approximately equal to half of the inside diameter). The serum of an HBs antigen-positive patient was diluted 4- to 64-fold with a stroma-containing phosphate buffer and a 0.25 ml portion of the dilution was added to the test tube containing the above-mentioned anti-HBs antibody-sensitized sheep erythrocyte suspension. After shaking, the tube was allowed to stand at room temperature for 3 hours. The same procedure was followed using a 320-fold dilution of a preparation containing 1 $\mu$ g/ml of purified HBs antigen as a standard HBs antigen. After 3 hours of standing, the diameter of an agglutinate appearing on the bottom of each test tube was measured using the apparatus according to the invention. The diameter was measured as the wave form width $\ell$ at the voltage value $V_{S.L.}$ which is sum value of $V_{S. MIN}$ and half of the difference between $V_{N. MAX}$ of the sensor output wave form for a negative control and $V_{S. MIN}$ of the sensor output wave form for the sample.

The diameters of the agglutination images of the patient's serum showed a linearity in the dilution range of 8 to 32 times.

Example 2

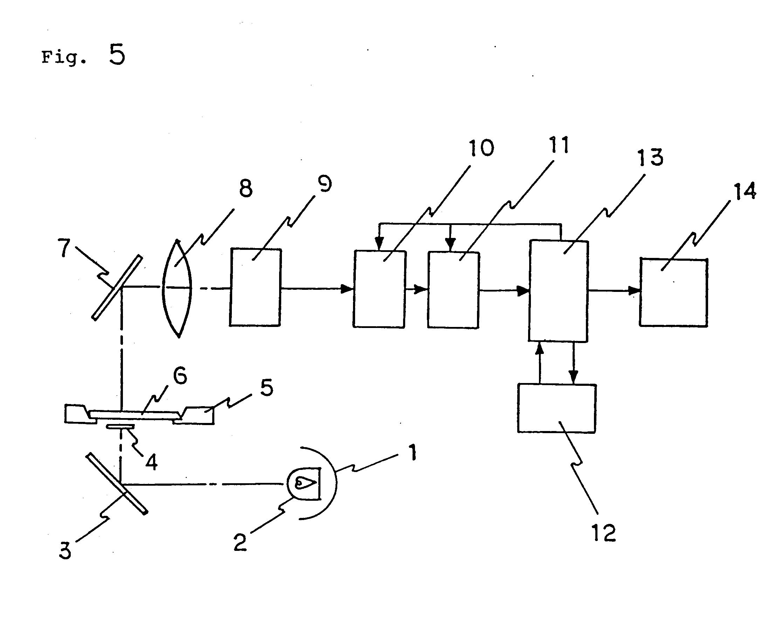

The apparatus shown in Fig. 5 was prepared. It comprises a reflector 1, a halogen lamp 2, a mirror 3, a diffusion plate for uniform light 4, a vessel-plate-supporting and -transferring frame 5, a sample-carrying plate 6, a mirror 7, an optical lens 8, a CCD linear image sensor 9, an A/D converter unit 10, a sensor wave form signal memory buffer 11, an operation panel unit 12, a wave form-processing computer unit 13, and a printer 14. The processing is carried out in the direction of arrows.

In accordance with the invention, rating and evaluation of agglutinates resulting from agglutination reactions can be made automatically. Thus, the diameter of the agglutinate is measured directly as a quantity correlative to the transverse area of the agglutinate which serves as the basis for rating, whereby it becomes possible to quantitatively detect a particular immune substance occurring in the sample. The method and apparatus provided by the invention make it possible to determine the agglutinate quantity objectively and quickly and are very useful in automatically detecting agglutination reactions in the field of diagnostic apparatus.

**Claims**

1. A method of quantitatively assaying an antigen or antibody in a sample, which comprises

(1) mixing the antigen- or antibody-containing sample and a support for agglutination reaction as sensitized with an antibody or antigen, respectively, corresponding to the antigen or antibody in said sample, in a reaction vessel to carry out an agglutination reaction, wherein the shape of the reaction vessel bottom is a spherical or conical one,

(2) allowing the agglutinate formed in step (1) to precipitate on the bottom of the reaction vessel to form an agglutinate precipitate,

(3) passing light from a light source through the reaction vessel from the bottom or the top thereof, thereby generating a particular intensity of the light after its passage through the agglutinate precipitate,

(4) continuously sensing the intensity of the transmitted light, on a line perpendicular to the direction of the light and passing through the center of the agglutinate precipitate, with a one-dimensional image sensor, and

(5) outputting the sensed result as a sensor output wave form (shown by voltage) and then measuring the diameter of the agglutinate precipitate in terms of the sensor output wave form width at a voltage intermediate between the standard voltage found with a negative control and the voltage found with the sample.

2. An apparatus suitable for quantitatively assaying an antigen or antibody in a sample in accordance with claim 1 which comprises

(a) a reaction vessel having a spherical or conical bottom for mixing the antigen- or antibody-containing sample and a support for agglutination reaction as sensitized with an antibody or antigen, respectively, corresponding to the antigen or antibody in said sample to carry out an agglutination reaction, and then allowing the agglutinate formed to precipitate on the bottom of said reaction vessel to form an agglutinate precipitate,

(b) a light source comprising a reflector (1) and a halogen lamp (2),

(c) a means for passing a light from said light source through the reaction vessel from the bottom or the top thereof, thereby generating a particular intensity of the light after its passage through the agglutinate precipitate, comprising a mirror (3), diffusion plate for uniform light (4), vessel-plate-supporting and -transferring frame (5) and a sample-carrying plate (6),

(d) a means for continuously sensing the intensity of the transmitted light on a line perpendicular to the direction of the light and passing through the center of the agglutinate precipitate with a one-dimensional image sensor, comprising a mirror (7), an optical lens (8) and a CCD linear image sensor (9),

(e) a means for measuring the diameter of the agglutinate precipitate by outputting the result sensed by said means (d) as a sensor wave form (shown by voltage) and in terms of the sensor output wave form width at a voltage intermediate between the standard voltage found with a negative control and the voltage found with the sample, comprising a A/D converter unit (10) and a sensor wave form signal memory buffer (11), and

(f) a means for calculating the size of the agglutinate precipitate by operation treatment of the value as measured by said means (e) for evaluation and judgment, comprising an operation panel unit (12), a wave form-processing computer unit (13) and a printer (14).

8

EP 0 198 327 B1

**Patentansprüche**

1. Methode zur quantitativen Bestimmung eines Antigens oder Antikörpers in einer Probe, welches umfaßt
(1) Mischen der Antigen- oder Antikörper-haltigen Probe und eines Trägers für die Agglutinationsreaktion, der mit einem Antikörper oder Antigen sensibilisiert worden ist, welche dem Antigen oder Antikörper in der Probe entsprechen, in einem Reaktionsgefäß, um eine Agglutinationsreaktion durchzuführen, wobei die Form des Reaktionsgefäßbodens kugelförmig oder konisch ist,
(2) Ausfällen des in Schritt (1) gebildeten Agglutinats am Boden des Reaktionsgefäßes, um einen Agglutinat-Niederschlag zu bilden,
(3) Durchleiten von Licht aus einer Lichtquelle durch das Reaktionsgefäß vom Boden zum oberen Ende, wodurch eine bestimmte Intensität des Lichts nach dessen Durchtritt durch den Agglutinat-Niederschlag erzeugt wird,
(4) kontinuierliches Abfühlen der Intensität des durchtretenden Lichtes auf einer Linie, die senkrecht zur Lichtrichtung und durch das Zentrum des Agglutinat-Niederschlags verläuft, mit einem eindimensionalen Bildsensor und
(5) Ausgeben des abgefühlten Ergebnisses als Sensor-Output-Wellenform (angezeigt als Spannung) und anschließendes Messen des Durchmessers des Agglutinat-Niederschlags anhand der Sensor-Output-Wellenformbreite bei einer Spannung zwischen der Standardspannung, die bei einer Negativkontrolle gefunden wird, und der Spannung, die bei der Probe gefunden wird.

2. Vorrichtung für die quantitative Bestimmung eines Antigens oder Antikörpers in einer Probe entsprechend Anspruch 1, welche umfaßt
(a) eine Reaktionsgefäß mit kugelförmigem oder konischem Boden zum Mischen der Antigen- oder Antikörper-haltigen Probe und eines Trägers für die Agglutinationsreaktion, der mit einem Antikörper oder Antigen sensibilisiert worden ist, welche dem Antigen oder Antikörper in der Probe entsprechen, um eine Aggutinationsreaktion durchzuführen, und anschließendes Ausfällen des gebildeten Niederschlages am Boden des Reaktionsgefäßes, um einen Agglutinat-Niederschlag zu bilden,
(b) eine Lichtquelle, umfassend einen Reflektor (1) und eine Halogenlampe (2),
(c) eine Einrichtung zum Durchleiten von Licht aus der Lichtquelle durch das Reaktionsgefäß vom Boden zum oberen Ende, wodurch eine bestimmte Intensität des Lichtes nach dessen Durchtritt durch den Aggutinat-Niederschlag erzeugt wird, welche umfaßt einen Spiegel (3), eine Diffusionsplatte für gleichmäßiges Licht (4), einen Gefäß-Platte tragenden und übertragenden Rahmen (5) und eine die Probe tragende Platte (6),
(d) eine Einrichtung zum kontinuierlichen Abfühlen der Intensität des durchtretenden Lichtes auf einer Linie, die senkrecht zur Lichtrichtung und durch das Zentrum des Agglutinat-Niederschlags verläuft, mit einem eindimensionalen Bildsensor, umfassend einen Spiegel (7), eine optische Linse (8) und einen linearen CCD-Bildsensor (9),
(e) eine Einrichtung zum Messen des Durchmessers des Agglutinat-Niederschlags durch Ausgeben des mit der Einrichtung (d) abgefühlten Ergebnisses als Sensor-Wellenform (angezeigt als Spannung) und anhand der Sensor-Output-Wellenformbreite bei einer Spannung zwischen der Standardspannung, die bei einer Negativkontrolle gefunden wird, und der Spannung, die bei der Probe gefunden wird, umfassend eine A/D-Wandlereinheit (10) und einen Sensor-Wellenfornsignal-Speicherpuffer (11) und
(f) eine Einrichtung zum Berechnen der Größe des Agglutinat-Niederschlags durch Operationsverarbeitung des von der Einrichtung (e) gemessenen Wertes für die Auswertung und Bewertung, umfassend eine Operationspulteinheit (12), eine Wellenform-Verarbeitungs-Computereinheit (13) und einen Drucker (14).

**Revendications**

1. Un procédé d'analyse quantitative d'un antigène ou d'un anticorps dans un échantillon, comprenant:
(1) le mélange de l'échantillon contenant un antigène ou un anticorps et d'un support de réaction d'agglutination sensibilisé, respectivement, par un anticorps ou un antigène, correspondant à l'antigène ou à l'anticorps dudit échantillon, dans un récipient de réaction pour effectuer une réaction d'agglutination, dans lequel la forme du fond du récipient de réaction est sphérique ou conique,
(2) la précipitation du mélange aggluté formé à l'étape (1) au fond du récipient de réaction pour former un précipité aggluté,

9

(3) l'émission d'une lumière produite par une source lumineuse à travers le récipient de réaction à partir du fond ou du haut de celui-ci, de manière que la lumière ait une intensité particulière après son passage à travers le précipité agglutiné,

(4) la détection continue de l'intensité de la lumière transmise, sur une ligne perpendiculaire à la direction de la lumière et passant par le centre du précipité agglutiné, à l'aide d'un capteur d'image unidimensionnel, et

(5) la sortie du résultat détecté sous forme d'un signal de sortie de capteur en forme d'onde (représenté par la tension) et la mesure du diamètre du précipité agglutine en fonction de la largeur de la forme d'onde du signai de sortie du capteur à une tension intermédiaire entre la tension normale détectée avec un témoin négatif et la tension trouvée avec l'échantillon.

2. Un appareil apte à effectuer l'analyse quantitative d'un antigène ou d'un anticorps dans un échantillon selon la revendication 1, comprenant:

(a) un récipient de réaction pourvu d'un fond sphérique ou conique pour mélanger l'échantillon contenant l'antigène ou l'anticorps et un support de réaction d'agglutination sensibilisé, respectivement, par un anticorps ou un antigène correspondant à l'antigène ou à l'anticorps dudit échantillon pour effectuer une réaction d'agglutination, et en laissant ensuite le mélange agglutiné ainsi formé se précipiter au fond dudit récipient de réaction pour former un précipité agglutiné,

(b) une source lumineuse comprenant un réflecteur (1) et une lampe à halogène (2),

(c) des moyens pour envoyer la lumière émise par ladite source lumineuse à travers le récipient de réaction, à partir du fond ou du haut de ce dernier, de manière à émettre une intensité lumineuse particulière après son passage à travers le précipité agglutiné, comprenant un miroir (3), une plaque de diffusion uniforme de la lumière (4), un châssis de support et de transport de récipient-plaque (5) et une plaque de support d'échantillon (6),

(d) des moyens pour détecter de façon continue l'intensité de la lumière transmise sur une ligne perpendiculaire à la direction de la lumière et passant par le centre du précipité agglutiné à l'aide d'un capteur d'image unidimensionnelle, comprenant un miroir (7), une lentille optique (8) et un capteur d'image linéaire à couplage de charge (9),

(e) des moyens pour mesurer le diamètre du précipité agglutiné,en sortant le résultat capté par lesdits moyens (d) sous forme d'un signal en forme d'onde émis par le capteur (exprimé par la tension) et en fonction de la largeur de la forme d'onde du signal de sortie du capteur a une tension intermédiaire, entre la tension standard obtenue avec un témoin négatif et la tension obtenue avec l'échantillon, comprenant un convertisseur analogique/numérique (10) et une memoire tampon de signal de capteur en forme d'onde (11), et

(f) des moyens pour calculer la dimension du précipité agglutiné par un traitement opératoire de la valeur mesurée par lesdits moyens (e) aux fins d'évaluation et de jugement, comprenant une unité de visualisation ou d'affichage (12), un calculateur de traitement de signaux (13) et une imprimante (14) en forme d'onde.

Fig. 1

Fig. 2

(a)

(b)

(c)

width

Fig. 3

(a)

[V]

$l_0$

$V_{N.MAX}$

Voltage

O

(b)

[V]

$l$

$V_{S.L}$
$V_{S.MIN}$

O

13

Fig. 4

Fig. 5